(19) Europäisches Patentamt
European Patent Office
Office européen des brevets

(11) **EP 4 717 275 A1**

(12) **EUROPEAN PATENT APPLICATION**
published in accordance with Art. 153(4) EPC

(43) Date of publication:
**01.04.2026 Bulletin 2026/14**

(21) Application number: **24806567.4**

(22) Date of filing: **14.05.2024**

(51) International Patent Classification (IPC):
***A61K 45/06*** *(2006.01)* ***A61K 31/4192*** *(2006.01)*
***A61K 31/506*** *(2006.01)* ***A61K 31/5377*** *(2006.01)*
***A61P 35/00*** *(2006.01)* ***A61P 35/04*** *(2006.01)*

(52) Cooperative Patent Classification (CPC):
**A61K 31/4192; A61K 31/506; A61K 31/5377; A61K 45/06; A61P 35/00; A61P 35/04**

(86) International application number:
**PCT/CN2024/093033**

(87) International publication number:
**WO 2024/235217 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.05.2023 CN 202310544888**

(71) Applicant: **Gunagdong Yinzhu Pharmaceutical Technology Co., Ltd**
**Foshan, Guangdong 528315 (CN)**

(72) Inventors:
- **CHI, Yansheng**
**Guangzhou, Guangdong 510530 (CN)**
- **HU, Shuanghua**
**Guangzhou, Guangdong 510530 (CN)**
- **XU, Kecheng**
**Guangzhou, Guangdong 510530 (CN)**
- **RONG, Yu**
**Guangzhou, Guangdong 510530 (CN)**
- **XING, Chengfeng**
**Guangzhou, Guangdong 510530 (CN)**
- **KOU, Yuhui**
**Guangzhou, Guangdong 510530 (CN)**
- **CHEN, Shijian**
**Guangzhou, Guangdong 510530 (CN)**

(74) Representative: **Winter, Brandl - Partnerschaft mbB**
**Alois-Steinecker-Straße 22**
**85354 Freising (DE)**

# (54) ANTI-LUNG CANCER COMBINED DRUG AND USE THEREOF

(57) Provided is an anti-lung cancer combined drug, which comprises carboxyamidotriazole and an anti-tumor targeting drug for gene mutation and is mainly applied to the preparation of anti-lung cancer drugs. Specifically, a combined administration group of carboxyamidotriazole and osimertinib can significantly inhibit the growth and proliferation of a human lung cancer NCI-H1975 xenograft tumor in a nude mouse, and a combined administration group of carboxyamidotriazole and alectinib can significantly inhibit the growth and proliferation of NCI-H2228 cells. The combined administration of the carboxyamidotriazole and the anti-tumor targeting drug for gene mutation has a combined synergistic effect and does not have obvious toxic and side effects.

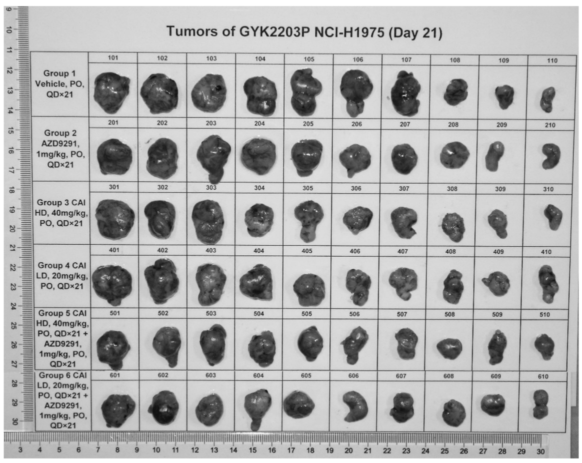


FIG. 6

EP 4 717 275 A1

## Description

### Technical Field

The present invention belongs to the field of medicine and healthcare. Specifically, it relates to a combination drug for treating lung cancers and use thereof.

### Background Art

Carboxyamidotriazole (CAI) is a non-cytotoxic antitumor drug. Its chemical name is 5-Amino-1-[3,5-dichloro-4-(4-chlorobenzoyl chloride)benzyl]-1H-1,2,3-triazole-4-benzamide, with the chemical structural formula:

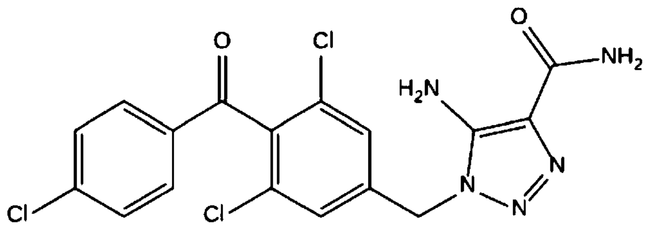

;

Current research indicates that CAI demonstrates promising efficacy against multiple tumor types. CAI inhibits the proliferation of endothelial cells and various tumor cell lines such as MDA-MB-231, OVCAR-3, and MCF-7. Additionally, it suppresses the invasion of prostate cancer, bladder cancer, breast cancer, liver cancer, glioblastoma, and others. Clinical trials reveal that CAI exhibits a mild antitumor effect, making it suitable for exploration in combination antitumor experiments.

Preliminary research on CAI's antitumor mechanisms and combination therapies has revealed synergistic effects when CAI is combined with sorafenib. Results indicate that the combination of CAI and sorafenib synergistically inhibits the growth of non-small cell lung cancer, a finding confirmed in both in vivo and in vitro experiments. This synergistic effect may be mediated by inducing apoptosis and suppressing NANOG expression. This combination significantly reduces the required sorafenib dosage in vivo without diminishing efficacy, while effectively preserving mouse body weight.

Collectively, these findings indicate substantial development potential for carboxamidazole-based combination therapies.

Chinese patent Application CN200810212286.4 discloses an antineoplastic drug formulation containing carboxamidazole and dexamethasone as active ingredients. In vivo animal studies showed that carboxamidazole alone achieved a 39.8% inhibition rate against mouse melanoma, while intraperitoneal dexamethasone injection yielded a 19.3% inhibition rate. The combined treatment achieved a 69.0% inhibition rate against transplanted melanoma tumors. Compared to the control group, both differences were highly significant.

Chinese patent application CN109674788A discloses the use of carboxamidazole combined with IDO1 inhibitors for antitumor applications. Specifically, the invention concerns the use of carboxamidazole in combination with one or more IDO1 inhibitors for preparing antitumor drugs in mammals. Despite continuous advances in antitumor drug research, the quest for more effective medications and approaches persists. Achieving complete eradication of tumors and eliminating cancer cells in clinical patients remains a goal pursued by industry professionals and anticipated by society.

Targeted drugs are specific agents designed to selectively kill cancer cells without affecting normal cells, thereby enhancing therapeutic efficacy. Take osimertinib as an example. its mechanism of action involves : lung cancer (currently limited to lung cancer, not applicable to all tumor types), which exhibits various genetic mutations. Approximately 50% of cases involve EGFR mutations. Research indicates that the carcinogenic process (the transformation of normal cells into tumor cells) in this type is driven by EGFR gene mutations. Osimertinib is a drug specifically developed to target the EGFR protein. Therefore, genetic testing is used to determine which targeted therapy to administer, identifying the specific target on the cancer cells to ensure precise treatment. For instance, in lung cancer, Patients exhibit mutations in certain genes, most commonly in the epidermal growth factor receptor (EGFR), anaplastic lymphoma kinase (ALK), and receptor tyrosine kinase 1 (ROS1). Different mutations require specific targeted therapies. However, existing targeted drugs face challenges of rapid drug resistance development.

Therefore, based on the above, exploring the combination of carboxamidazole with anti-tumor targeted drugs against specific gene mutations aims to reduce drug resistance, increase drug sensitivity, and achieve synergistic effects. This approach can benefit more patients.

**Summary**

To address the aforementioned issues, the present invention provides a combination drug for treating lung cancer. It achieves synergistic effects by combining carboxamidazole with mutation-targeted anticancer drugs, while exhibiting no significant toxic side effects.

To accomplish the above objectives, the present invention employs the following technical proposal:
A combination drug for anti-lung-cancer, which comprises a carboxyamidotriazole and an antitumor targeted drug for gene mutations.

Preferably, the antitumor targeted drug is selected from one or more of an antitumor targeted drug for EGFR mutations, an antitumor targeted drug for ALK rearrangements, an antitumor targeted drug for MET mutations, an antitumor targeted drug for ROS1 rearrangements, an antitumor targeted drug for BRAFV600 mutations and an antitumor targeted drug for RET fusions.

Further preferably, wherein the antitumor targeted drug for EGFR mutations is selected from at least one of an osimertinib, an icotinib, a gefitinib, an afatinib, an almonertinib or a dacomitinib.

Further preferably, the antitumor targeted drug for ALK rearrangements is selected from at least one of a crizotinib, an alectinib, a ceritinib, an ensartinib or a brigatinib.

Further preferably, the antitumor targeted drug for MET mutations is savolitinib; the antitumor targeted drug for ROS1 rearrangements is selected from at least one of a crizotinib or an entrectinib; the antitumor targeted drug for BRAFV600 mutations is selected from at least one of a dabrafenib or a trametinib; the antitumor targeted drug for RET fusions is pralsetinib.

More preferably, the combination drug comprises the carboxyamidotriazole and the osimertinib.

More preferably, the combination drug comprises the carboxyamidotriazole and the alectinib.

Preferably, for a subject, the dosage of the carboxyamidotriazole is 1-10mg/kg; the dosage of the antitumor targeting drug is 0.05-10mg/kg.

Preferably, the dosage of the carboxyamidotriazole is 1-4mg/kg; the dosage of the antitumor targeting drug is 0.05-0.1mg/kg.

Preferably, the effective daily dosage of the carboxyamidotriazole is 50-300mg; the dosage of the antitumor targeting drug is 2mg-1200mg/day.

Further preferably, the effective daily dosage of the carboxyamidotriazole is 150-300mg; the dosage of the antitumor targeting drug is 150mg-600mg/day.

Preferably, the effective daily dosage of the carboxyamidotriazole is 25-195mg, preferably 50-150mg/day; the dosage for a person estimated from animal studies is about 1-4mg/kg, preferably 1.626-3.252mg/kg; the dosage of osimertinib for a person estimated from animal studies is about 0.05-0.1mg/kg, preferably 0.0813mg/kg.

Preferably, the effective daily dosage of alectinib which ranges from 600-1560 mg/day; in some embodiments within the scope of the present invention, it may be 1200 mg/day or 840 mg/day; the effective daily dosage of crizotinib which ranges from 125-325 mg/day; in some embodiments within the scope of the present invention, it may be 250 mg/day; the effective daily dosage of ceritinib which ranges from 225-585 mg/day; in some embodiments within the scope of the present invention, it may be 1200 mg/day or 450 mg/day; the effective daily dosage of ensartinib which ranges from 112-300 mg/day; in some embodiments within the scope of the present invention, it may be 225 mg/day; the effective daily dosage of brigatinib which ranges from 45-240 mg/day; in some embodiments within the scope of the present invention, it may be 45-180 mg/day, in some embodiments within the scope of the present invention, it may be 90-180 mg/day.

Preferably, the effective daily dosage of osimertinib is 40-104mg/day, preferably 56mg/day; the effective daily dosage of icotinib is 187.5-487.5mg/kg; preferably 245.5mg/day; the effective daily dosage of gefitinib is 125-325mg/day; in some embodiments within the scope of the present invention, it may be 250mg/day; the effective daily dosage of afatinib is 20-52mg/day; preferably 28mg/day; the effective daily dosage of almonertinib is 55-143mg/day; preferably 77mg/day; the effective daily dosage of dacomitinib is 22.5-58.5mg/day; preferably 31.5mg/day; the effective daily dosage of savolitinib is 200-780mg/day; preferably 180-420mg/day; the effective daily dosage of entrectinib is 300-780mg/day; preferably 420mg/day; the effective daily dosage of dabrafenib is 150-390mg/day; preferably 210mg/day; the effective daily dosage of trametinib is 1-2.6mg/day; preferably 1.4mg/day; the effective daily dosage of pralsetinib is 200-520mg/day; preferably 280mg/day.

Preferably, the combination drug comprises by weight portion, 20-40 portions of carboxyamidotriazole and 0.5-1.5 portions of osimertinib.

Preferably, the dosage form of carboxyamidotriazole is softgel capsules, the dosage form of osimertinib is tablets.

Preferably, the carboxyamidotriazole and the antitumor drug targeting gene mutations may be administered simultaneously, separately or sequentially.

The present invention also relates to a use of the combination drug for preparing anti-lung cancer drug.

Preferably, the function of the combination drug comprises one or more of reducing a tumor volume, increasing a

tumor growth inhibition rate, decreasing a relative tumor volume, decreasing relative tumor proliferation rate, or increasing a tumor inhibition rate.

The present invention also relates to a composition, which comprises the abovementioned combination drug, that is, the carboxyamidotriazole and the antitumor drug targeting gene mutations, and the composition is used for preparation of anti-lung-cancer drug.

Preferably, the composition comprises a pharmaceutically acceptable vehicle or excipient.

Preferably, the dosage form of the composition is selected from any one of oral liquids, softgel capsules, oral suspensions, solid dispersions, capsules, sustained-release formulations, granules, tablets, injections, ointments, patches, or implants.

Compared with prior art, the present invention provides the following beneficial effects:

(1) The combination of carboxyamidotriazole and osimertinib significantly inhibited the growth and proliferation of human lung cancer NCI-H1975 xenografts in nude mice *(P<0.01, P<0.01).* The combination of two drugs exhibited synergistic effects (*P<0.05, P<0.05,* synergy index >1.15).

(2) The combination of carboxyamidotriazole and osimertinib did not inhibit animal weight gain. Compared to Day 0 at the start of the experiment, the average animal weight increased by 8.51%-11.04% (1.63 g-2.17 g). No drug-related animal deaths or other significant drug-related toxic side effects were observed during the experiment.

(3) Carboxyamidotriazole demonstrated significant synergistic effects when combined with targeted therapies for: targeted drugs for EGFR mutations, targeted drugs for ALK rearrangements, targeted drugs for MET mutations, targeted drugs for ROS1 gene rearrangements, targeted drugs for BRAFV600 mutations, and targeted drugs for RET fusions.

(4) The combination of carboxyamidotriazole and alectinib significantly inhibited the growth and tumor proliferation of human lung adenocarcinoma NCI-H2228 cells, demonstrating a marked synergistic effect.

## Brief Description Of The Drawings

FIG. 1 is a curve chart of animal body weight for Comparative Examples 1-3 and Examples 1-2;
FIG. 2 is a curve chart of rate of animal body weight change for Comparative Examples 1-3 and Examples 1-2;
FIG. 3 is a curve chart of tumor volume for Comparative Examples 1-3 and Examples 1-2;
FIG. 4 is a curve chart of relative tumor volume for Comparative Examples 1-3 and Examples 1-2;
FIG. 5 is a bar chart of tumor weight for Comparative Examples 1-3 and Examples 1-2;
FIG. 6 is a comparison of photograph of tumor for Comparative Examples 1-3 and Examples 1-2;
FIG. 7 is a curve chart of tumor volume for Comparative Examples 4-6 and Examples 4-5;
FIG. 8 is a curve chart of relative tumor volume for Comparative Examples 4-6 and Examples 4-5;
FIG. 9 a bar chart of tumor weight for Comparative Examples 4-6 and Examples 4-5.

## Detailed Description

The following detailed description of the present invention is provided with reference to specific embodiments. These embodiments are not intended to limit the scope of the invention but merely serve to illustrate it. Unless otherwise specified, the experimental methods used in the following embodiments, where specific conditions are not noted, are typically performed under conventional conditions. Unless otherwise specified, the materials, reagents, and other items used in the following embodiments are commercially available.

Carboxyamidotriazole (CAI), osimertinib (AZD9291) and alectinib hydrogen chloride are provided by Guangdong Yinzhu Pharmaceutical Technology CO., LTD). Detailed information is shown in Table 1.

Table 1 Drugs and its related information

| Drugs | Batch Number | Purity | Molecular weight (g/mol) | Storage |
|---|---|---|---|---|
| Carboxyamidotriazole (CAI) softgel capsule | 69921110802 | 99.8% | / | 4 °C, in dark |
| Osimertinib (AZD9291) Tablets | S729709 | >95% | 499.61 | 4 °C, in dark |
| Alectinib hydrogen chloride | HY-13011A | 99.91 | 519.08 | 4 °C, in dark |

Example 1

A combination drug for treating lung cancers, comprises by weight portion: 40 portions of carboxyamidotriazole (PO, QD×21), 1 portion of osimertinib (PO, QD×21).

Example 2

A combination drug for treating lung cancers, comprises by weight portion: 20 portions of carboxyamidotriazole (PO, QD×21), 1 portion of osimertinib (PO, QD×21).

| | |
|---|---|
| Comparative Example 1 | 1 portion of osimertinib (PO, QD×21). |
| Comparative Example 2 | 40 portions of carboxyamidotriazole (PO, QD×21). |
| Comparative Example 3 | 20 portions of carboxyamidotriazole (PO, QD×21). |

Control Group

Solvent control group, solvent as a vehicle (PO, QD×21) without carboxyamidotriazole or osimertinib.

A. Evaluation of the therapeutic effect of the drugs in the abovementioned Examples 1-2 and Comparative Examples 1-3:

1. Animals used in the experiment

1.1 Animal information

BALB/c Nude mice, female, 8-10 weeks old, 18-20g. The variation of the body weight is ±20% around average body weight. 80 mice were inoculated and 60 mice were grouped.

Animal Source: Jiangsu Jicui Yao Kang Biotechnology Co., Ltd., Production License No.: SCXK(Su)2018-0008, Animal Compliance Certificate No.: 202301350.

1.2 Animal Housing

SPF environment, IVC mouse cages, 5 mice per cage. Temperature: 20-26°C, Humidity: 40-70%, Lighting: 12-hour light-dark cycle. Feed: Irradiated rodent feed purchased from Beijing Ke'ao Xie Li Feed Co., Ltd., ad libitum.

Drinking water: Municipal tap water, filtered and autoclaved before use.

Bedding: Corn cobs purchased from Ke'ao Xie Li, autoclaved before use and replaced weekly.

Acclimatization period: Mice underwent a minimum 7-day acclimatization period prior to experiments.

Animal identification: Each cage displayed an information tag containing mouse details, cell inoculation data, animal experiment information, and experimenter details. Mice were marked using ear tags.

1.3 Experimental Animal Management Guidelines

All experimental animals are housed in the SPF-grade animal facility of Shanghai Medix Biopharmaceutical Co., Ltd. during the study. Daily care and experimental procedures are conducted by the animal care staff of Shanghai Medix Biopharmaceutical Co., Ltd.

All animal handling and management strictly adhere to the Shanghai Medix Guidelines for the Use and Management of Laboratory Animals.

2. Experimental Materials

2.1 Solvent Formulations and Storage Conditions for Administered Solutions

2.1.1 CAI

Solvent: 100% PEG400
Storage Conditions for Administered Solution: Store at room temperature protected from light. Shelf life: 3 days.

2.1.2 AZD9291

Solvent: 0.5% MC
Storage Conditions for Administered Solution: Store at 4°C protected from light. Shelf life: 7 days.

2.2 Cell Lines

Human lung cancer cell line NCI-H1975 was purchased from the American Type Culture Collection (ATCC, Manassas, VA, USA).

2.3 Media

RPMI 1640 basic medium and fetal bovine serum (FBS) were purchased from GIBCO (Grand Island, NY, USA). Matrigel was purchased from Corning (Corning, NY, USA).

3. Experimental Design

The experimental design is shown in Table 2.

Table 2 Experimental design of therapeutic effect of the drugs in vivo

| Groups | Drug | Animal group capacity | Dosage (mg/kg ) | Dosage in concentration (mg/mL) | Dosage in volume (mL/kg ) | Route of administration | Timetable of administration |
|---|---|---|---|---|---|---|---|
| Control Group | Vehicle | 10 | NA | NA | 10 | PO | QD×21 |
| Comparative Example 1 | AZD9291 | 10 | 1 | 0.1 | 10 | PO | QD×21 |
| Comparative Example 2 | CAI | 10 | 40 | 4 | 10 | PO | QD×21 |
| Comparative Example 3 | CAI | 10 | 20 | 2 | 10 | PO | QD×21 |
| Example 1 | CAI | 10 | 40 | 4 | 10 | PO | QD×21 |
| | AZD929 1 | | 1 | 0.1 | 10 | PO | QD×21 |
| Example 2 | CAI | 10 | 20 | 2 | 10 | PO | QD×21 |
| | AZD929 1 | | 1 | 0.1 | 10 | PO | QD×21 |

4. Experimental Methods

4.1 Model Establishment

NCI-H1975 cells were cultured in RPMI 1640 medium supplemented with 10% FBS and maintained in a 37°C, 5% $CO_2$ humidified incubator.

Log-phase NCI-H1975 cells were harvested, resuspended in RPMI 1640 basal medium containing 50% Matrigel, and adjusted to a concentration of $2 \times 10^7$ cells/mL. Under aseptic conditions, 0.1 mL of the cell suspension was injected subcutaneously into the right flank of mice at a concentration of $2 \times 10^6$ cells/0.1 mL/mouse.

4.2 Grouping and Administration

When the mean tumor volume reached approximately 150 $mm^3$, animals were randomly grouped based on tumor volume, ensuring inter-group tumor volume variation was less than 10% of the mean.
The grouping day was designated as Day 0, and administration commenced according to animal body weight. During dosing, individual animals exhibiting a body weight loss exceeding 15% compared to Day 0 (BWL ≥ 15%) will be discontinued from treatment until weight recovery (BWL < 15%), after which dosing will resume.

4.3 Weighing and Observation

Animal body weight and tumor volume were measured twice weekly throughout the experiment. Tumor length and width were measured using a digital caliper, with tumor volume estimated from these measurements.
Clinical observations were recorded daily to assess overall health status, weight abnormalities, behavioral changes, and other treatment-related adverse reactions.

4.4 Endpoint Criteria

In accordance with animal welfare regulations, animals meeting any of the following criteria during the study were removed from the experimental group and euthanized: 1. Body weight loss exceeding 20% compared to Day 0 (BWL ≥ 20%); 2. Severe adverse reactions such as blindness or paralysis; 3. Tumor volume exceeding 2000 $mm^3$; 4.
Formation of open ulcers on the tumor surface.
After the final weighing on Day 21, remaining animals were euthanized with $CO_2$. Tumors were weighed, photographed, and the experiment concluded.

4.5 Evaluation Indicators

Tumor volume (TV) is calculated as: $1/2 \times a \times b^2$, where a and b represent the measured length and width of the tumor, respectively.
Tumor growth inhibition rate ($\%TGI_{TV}$) is calculated as: $(1 - TV_T/TV_C) \times 100\%$, where TVc denotes the mean tumor volume of the negative control group and $TV_T$ denotes the mean tumor volume of the treatment group.
Relative Tumor Volume (RTV) is calculated as $Vt/V_0$, where $V_0$ is the tumor volume at grouping and Vt is the tumor volume at each measurement.
Relative Tumor Growth Rate ($\%T/C_{RTV}$) is calculated as $T_{RTV}/C_{RTV} \times 100\%$, where $T_{RTV}$ is the RTV of the treatment group and $C_{RTV}$ is the RTV of the negative control group.
Tumor growth inhibition rate ($\%TGI_{TW}$) is calculated as: $(TWc - TW_T) / TWc \times 100\%$, where TWc is the average tumor weight in the negative control group and $TW_T$ is the average tumor weight in the treatment group.
The animal body weight change rate (%BWC) is calculated as: $(BW_t - BW_0) / BW_0 \times 100\%$, where $BW_t$ is the animal body weight at each measurement and $BW_0$ is the animal body weight at grouping.
According to China's NMPA Technical Guidance Principles for Non-clinical Studies of Cytotoxic Antineoplastic Drugs (November 2006), $\%T/C_{RTV} \leq 40\%$ and statistically significant *P < 0.05* indicate efficacy. Drug-related animal mortality exceeding 20% indicates severe toxicity at that dose.

5. Statistical Analysis

All experimental data in this study are expressed as Mean ± SEM.
Intergroup comparisons were performed using two-tailed t-tests. *P < 0.05* was considered statistically significant, and *P < 0.01* was considered highly significant (Microsoft Excel 2007, Redmond, WA, USA).

6. Experimental Results

Influence of animal body weight and fatality for different combination of drugs is shown in Table 3.

Table 3 Influence of animal body weight for different combination of drugs

| Groups | Animal group capacity | Drugs | Average body weight (g, Mean ± SEM) | | Body weight change rate Day 21 vs Day 0 | Fatality |
|---|---|---|---|---|---|---|
| | | | Day 0 | Day 21 | | |
| Control group | 10 | Vehicle, PO, QD×21 | 19.60 ± 0.25 | 21.77 ± 0.35 | 11.04% (2.17 g) | 0/10 (Day 21) |
| Comparative Example 1 | 10 | AZD9291, 1mg/kg, PO, QD×21 | 19.47 ± 0.28 | 21.46 ± 0.42 | 10.15% (1.99 g) | 0/10 (Day 21) |
| Comparative Example 2 | 10 | CAI, 40mg/kg, PO, QD×21 | 19.49 ± 0.20 | 21.58 ± 0.46 | 10.78% (2.09 g) | 0/10 (Day 21) |
| Comparative Example 3 | 10 | CAI, 20mg/kg, PO, QD×21 | 19.37 ± 0.36 | 21.19 ± 0.35 | 9.61% (1.82 g) | 0/10 (Day 21) |
| Example 1 | 10 | CAI, 40mg/kg, PO, QD×21 + AZD9291, 1mg/kg, PO, QD×21 | 19.48 ± 0.27 | 21.62 ± 0.33 | 10.99% (2.14 g) | 0/10 (Day 21) |
| Example 2 | 10 | CAI, 20mg/kg, PO, QD×21 + AZD9291, 1mg/kg, PO, QD×21 | 19.49 ± 0.31 | 21.12 ± 0.23 | 8.51% (1.63 g) | 0/10 (Day 21) |

As shown in Table 3, no significant inhibitory effect on animal weight gain was observed in the administration of single components AZD9291/CAI in Comparative Examples 1-3 and the administration of combination drugs in Examples 1-2, towards the solvent control group. The weight change curves are depicted in Figure 1. Compared to Day 0 at the start of the experiment, the average animal body weight increased by 8.51%-11.04% (1.63 g-2.17 g). The body weight change rate curve is shown in Figure 2. No drug-related animal deaths or other significant drug-related toxic side effects were observed during the experiment.

Influence on tumor volume, tumor weight and tumor inhibition rate for different combination of drug is shown in Table 4.

Table 4 Influence on tumor volume, tumor weight and tumor inhibition rate for different combination of drugs.

| Groups | Ani mal grou p capa city | Drugs | Tumor volume ($mm^3$,Mean±SE M) | | Tum or grow th inhib ition rate( %) Day 21 | Relati ve tumor volu me Day 21 | Relativ e tumor prolife ration rate(%) Day 21 | Tumor weight (g,Mean ±SEM) Day 21 | Tum or inhib ition rate( %) Day 21 |
|---|---|---|---|---|---|---|---|---|---|
| | | | Day 0 | Day 21 | | | | | |
| Contro 1 group | 10 | Vehicle, PO, QD×21 | 158. 42 ± 16.1 8 | 1765.54± 270.49 | / | 10.78 ± 1.14 | / | 1.643± 0.250 | / |
| Compa rative Examp le 1 | 10 | AZD9291,1 mg/kg, PO, QD×21 | 158. 00 ± 17.1 2 | 1394.40 ±181.52 | 21.02 | 8.74 ± 0.73 | 81.08 | 1.401± 0.212 | 14.73 |
| Compa rative Examp le 2 | 10 | CAI, 40mg/kg,PO, QD×21 | 158. 34 ± 17.5 1 | 1536.24 ±222.57 | 12.99 | 10.03 +1.27 | 93.04 | 1.384± 0.266 | 15.76 |
| Compa rative Examp le 3 | 10 | CAI, 20mg/kg,PO, QD×21 | 159. 13 ± 16.5 9 | 1547.91 ±221.67 | 12.33 | 10.05 ±1.30 | 93.23 | 1.527± 0.257 | 7.06 |

(continued)

| Groups | Animal group capacity | Drugs | Tumor volume (mm³,Mean±SE M) | | Tumor growth inhibition rate( %) Day 21 | Relative tumor volume Day 21 | Relative tumor proliferation rate(%) Day 21 | Tumor weight (g,Mean ±SEM) Day 21 | Tumor inhibition rate( %) Day 21 |
|---|---|---|---|---|---|---|---|---|---|
| | | | Day 0 | Day 21 | | | | | |
| Example 1 | 10 | CAI, 40mg/kg,PO, QD×21 + AZD9291,1 mg/kg, PO, QD×21 | 159. 20 ± 15.5 4 | 1066.90 ±120.43* | 39.57 #& | 6.72± 0.42** #& | 62.34 | 1.087± 0.167*#& | 33.84 |
| Example 2 | 10 | CAI,20mg/kg, PO, QD×21 + AZD9291,1 mg/kg,PO, QD×21 | 158. 95 ±15. 90 | 1127.09 ±130.54* | 36.16 #※ | 7.12± 0.54** #※ | 66.05 | 1.090± 0.139*#※ | 33.66 |

Compared to control group, *P<0.05, **P<0.01; compared to Comparative Example 1, #P<0.05; compared to Comparative Example 2, &P<0.05; compared to Comparative Example 3, ※P<0.05.

The evaluation of the combination index (CI) of drugs relies on the following equation: CI=E(A+B)/(EA+EB-EA×EB), wherein E(A+B) is the inhibition rate of combination drug, EA and EB is the inhibition rate of drug A and drug B respectively. E(A+B) in the equation represents 'measured synergistic effect' and (EA+EB-EA×EB) is 'expected synergistic effect'. CI=0.85-1.15 indicates a mere addition of the effect of two drugs, >1.15 indicates enhancement (or synergy), and <0.85 indicates antagonism.

Combination index of tumor growth inhibition: CAI, 40mg/kg + AZD9291, 1mg/kg: 1.27;
CAI, 20mg/kg + AZD9291, 1mg/kg: 1.18;

Combination index of tumor inhibition: CAI, 40mg/kg + AZD9291, 1mg/kg: 1.20;
CAI, 20mg/kg + AZD9291, 1mg/kg: 1.62.

Based on the tumor volume, weight, and tumor inhibition rate data in Table 4, as well as the comparative curves of tumor volume, relative tumor volume, and tumor weight shown in Figures 3 to 5, it can be observed that in Comparative Example 1, the single component AZD9291 administered at a dose of 1 mg/kg alone resulted in an average tumor weight of 1.401±0.212 g and a tumor inhibition rate %$TGI_{TW}$ of 14.73%. It shows no significant difference compared to the average tumor weight (1.643±0.250 g) in the solvent control group (P > 0.05). The relative tumor proliferation rate %$T/C_{RTV}$ was 81.08% (P > 0.05).

In Comparative Example 2, the average tumor weight for the single component CAI administered alone at 40 mg/kg was 1.384 ± 0.266 g, and in Comparative Example 3, it was 1.527 ± 0.257 g when administered alone at 20 mg/kg. The tumor inhibition rate % $TGI_{TW}$ were 15.76% and 7.06%, respectively, showing no significant difference compared to the solvent control group (P>0.05, P>0.05); the relative tumor proliferation rates %$T/C_{RTV}$ were 93.04% (P > 0.05) and 93.23% (P > 0.05), respectively.

In Example 1, the combination of CAI at 40 mg/kg and AZD9291 at 1 mg/kg resulted in an average tumor weight of 1.087±0.167 g and a tumor inhibition rate %$TGI_{TW}$ of 33.84%, showing significant differences compared to the average tumor weights in Comparative Examples 1-2 and the solvent control group (P < 0.05). The relative tumor growth rate % $T/C_{RTV}$ was 62.34% (P<0.01), showing significant difference compared to the administration of AZD9291 1 mg/kg alone in Comparative Example 1 (P<0.05), and a significant difference compared to the administration of CAI 40 mg/kg alone in Comparative Example 2 (P<0.05).

In Example 2, the combination of CAI 20 mg/kg and AZD9291 1 mg/kg resulted in an average tumor weight of 1.090 ± 0.139 g and a tumor inhibition rate %$TGI_{TW}$ of 33.66%, showing significant differences compared to the average tumor weights in Comparative Examples 1, 3, and the solvent control group (P < 0.05). The relative tumor growth rate % $T/C_{RTV}$ was 66.05% (P<0.01), showing significant differences compared to the administration of AZD9291 1 mg/kg alone in Comparative Example 1 (P<0.05) and the administration of CAI 20 mg/kg alone in Comparative Example 3 (P<0.05). According to the comparison of photograph of tumor shown in FIG. 6 and above data, it indicates that the combination of

CAI 40 mg/kg and AZD9291 1 mg/kg in Example 1, as well as the combination of CAI 20 mg/kg and AZD9291 1 mg/kg in Example 2, significantly inhibited the growth and proliferation of human lung cancer NCI-H1975 xenografts in nude mice (P<0.01, P<0.01). Combined with the evaluation of the combination index of drug, the combination of CAI and AZD9291 in Examples 1-2 demonstrated an obvious synergistic effect.

Example 3 Evaluation of the cellular therapeutic effect of the combination of carboxamidazole and alectinib *in vitro*

1. Research methods

CCK8 method was used to evaluate the effect of the drugs towards proliferation of lung cancer cell line (NCI-H2228 cell). The cellular viability with different concentration of drug was assayed and the 50% inhibitory concentration was calculated.

2. Drugs and main reagents

The drugs and reagents used in the evaluation of the cellular therapeutic effect are shown in Table 5-6.

Table 5

| Name | Purity(%) | Solvent | Preservation | Preservation after dissolution |
|---|---|---|---|---|
| Carboxyamidotriazole | 99.8 | DMSO | -20°C | -20°C |
| Alectinib | 99.91 | DMSO | 4°C | -80°C |

Table 6

| Name | Batch number | Preservation | Expiration date | Manufacturer |
|---|---|---|---|---|
| 1640 cultural medium | 2717598 | 4°C | 2024.09 | gibco |
| Fetal bovine serum | 7-3401-B | -20°C | 2026.04 | Chiscientific. biology |
| Trypsin | 20001A | 4°C | 2024.10 | gibco |
| PBS buffer | 8123354 | 4°C | 2025.05 | gibco |
| CCK8 | HY-K0301 | 4°C | 2025.01 | MCE |

3. Experimental Methods

3.1 Cell Thawing

Emerge NCI-H2228 cells (purchased from American Type Culture Collection (ATCC, Manassas, VA, USA)) from liquid nitrogen and thaw in a 37°C water bath. After thawing, culture cells in 1640 medium.

3.2 Cell Passaging and Plating

When cell density reached 90% confluence in culture medium, cells were digested with 0.25% trypsin and resuspended. After cell growth stabilized, cells in the logarithmic growth phase were harvested and counted using a cell counter and it was ensured that viability exceeded 85% for all cell lines.

Adjust cell concentration by diluting with culture medium. Add 100 μL of cell suspension to a 96-well plate to achieve a density of 3000 cells/well. Add 100 μL of PBS buffer to the wells around the cell-seeded wells.

Incubate the 96-well plate at 37°C, 5% $CO_2$, and 95% humidity overnight.

3.3 Cell Adding samples

Remove the cell culture plate from the incubator, aspirate the culture medium, and add the prepared drug-containing medium. Incubate at 37°C, 5% $CO_2$, and 95% humidity for 72 hours.

3.4 CCK-8 Assay

Add 10 μL of CCK-8 solution to each well of the 96-well plate. Incubate the plate in the cell culture incubator for 1.5 hours. Measure the absorbance at 450 nm using a microplate reader.

3.5 Data Analysis Method

Analyze data using GraphPad Prism 5.0 software. Fit the data using nonlinear S-curve regression to generate a dose-response curve and calculate the IC50 value.

Cell survival rate (%) = (OD test drug - OD media control) / (OD cell control - OD media control) × 100%.

The analysis of the combination index of the compound was performed using the median effect principle. The formula for the combination index of two compounds is:

$$CI=(D)1/(Dx)1+(D)2/(Dx)2$$

Where:

(D)1---- The concentration of Compound 1 when compound 1 was used with another compound and the effect X was generated;
(D)2---- The concentration of Compound 2 when compound 2 was used with another compound and the effect X was generated;
(Dx)1---- The concentration of Compound 1 when compound 1 was used alone and the effect X was generated;
(Dx)2---- The concentration of Compound 2 when compound 2 was used alone and the effect X was generated.

CI value quantitative judgment

The CI value enables quantitative assessment of the nature and strength of interactions between different compounds (Table 2). Interaction strength is measured using the weighted average CI (CIwt), calculated by the formula:

$$CIwt = (CI50 + 2CI75 + 3CI90 + 4CI95) / 10$$

Where:

CI50 ---- CI value at EC50 (concentration achieving 50% effect)
CI75, CI90, and CI95 follow the same principle, representing the CI values at EC75, EC90, and EC95, respectively.

3.6 Judgment criteria of the results

Evaluation of the combination effect: CI value is used to evaluate the combination effect, and the judgment method is shown in Table 7.

Table 7 Judgment criteria of the results

| Range of CI value | Judgment criteria | Symbles |
|---|---|---|
| CIwt<0.1 | Very strong synergistic effect | + + + + + |
| 0.1<CIwt<0.3 | Strong synergistic effect | + + + + |
| 0.3<CIwt<0.7 | Fairly strong synergistic effect | + + + |
| 0.7<CIwt<0.85 | Medium synergistic effect | + + |
| 0.85<CIwt<0.90 | Weak synergistic effect | + |
| 0.90<CIwt< 1.10 | Mere addition | ± |
| 1.10<CIwt< 1.20 | Weak antagonistic effect | - |
| 1.20<CIwt<1.45 | Medium antagonistic effect | - - |
| 1.45<CIwt<3.3 | Fairly strong antagonistic effect | - - - |
| 3.3<CIwt<10 | Strong antagonistic effect | - - - - |

(continued)

| Range of CI value | Judgment criteria | Symbles |
|---|---|---|
| 10<CIwt | Very strong antagonistic effect | - - - - - |

4. Experimental results

4.1 Observation on cells

No sediment or crystal of drugs; NCI-H2228 exhibited good cell viability and normal cell morphology. No bacteria or fungus contamination.

4.2 Experimental results

The IC50 of CAI alone to NCI-H2228 cell line was 22.427,22.877,23.018 μM respectively in 3 plates, with an average of 22.774 μM; the IC50 of Alectinib alone to NCI-H2228 cell line was 0.563,0.471,0.546 μM respectively in 3 plates, with an average of 0.527μM. After the drug combination, the IC50 of CAI changed to 1.660,1.092,2.150 μM, with an average of 1.634 μM; the IC50 of Alectinib changed to 0.332,0.218,0.430 μM, with an average of 0.327μM. The combination index were 0.366,0.203,0.540 respectively, with an average of 0.370. The results demonstated that CAI showed a fairly strong synergistic effect (+++) in combination with Alectinib, which is shown in Table 8.

Table 8 Effect on cellular viability of NCI-H2228 by CAI in combination of Alectinib

| - | | IC50 (CAI,μM ) | IC50 (Alectinib, μM) | CAI+ Alectinib | | CI value | Judgment result |
|---|---|---|---|---|---|---|---|
| | | | | IC50 (CAI,μM) | IC50 Alectinib,μM) | | |
| NCI-H2228 | 1 | 22.427 | 0.563 | 1.660 | 0.332 | 0.366 | +++ Fairly strong synergistic effect |
| | 2 | 22.877 | 0.471 | 1.092 | 0.218 | 0.203 | |
| | 3 | 23.018 | 0.546 | 2.150 | 0.430 | 0.540 | |
| | Average | 22.774 | 0.527 | 1.634 | 0.327 | 0.370 | |

4.3 Conclusion

Under the experimental conditions, the results indicate that the combination of CAI and alectinib exhibits a strong synergistic effect in inhibiting the growth and proliferation of NCI-H2228 cells.

B. Evaluation of the therapeutic effect on animals of the combination of carboxamidazole and alectinib

1. Animals used in the experiment

1.1 Animal information

BALB/c Nude mice, female, 8-10 weeks old, 18-20g. The variation of the body weight is ±20% around average body weight. 80 mice were inoculated and 60 mice were grouped.

Animal Source: Jiangsu Jicui Yao Kang Biotechnology Co., Ltd., Production License No.: SCXK(Su)2018-0008, Animal Compliance Certificate No.: 202329657.

1.2 Animal Housing

Housing condition: SPF environment, IVC mouse cages, 5 mice per cage. Temperature: 20-26°C, Humidity: 40-70%, Lighting: 12-hour light-dark cycle. Feed: Irradiated rodent feed purchased from Beijing Ke'ao Xie Li Feed Co., Ltd., ad libitum. Drinking water: Municipal tap water, filtered and autoclaved before use. Bedding: Corn cobs purchased from Ke'ao Xie Li, autoclaved before use and replaced weekly.

Acclimatization period: Mice underwent a minimum 7-day acclimatization period prior to experiments.

Animal identification: Each cage displayed an information tag containing mouse details, cell inoculation data,

animal experiment information, and experimenter details. Mice were marked using ear tags.

1.3 Experimental Animal Management Guidelines

All experimental animals are housed in the SPF-grade animal facility of Shanghai Medix Biopharmaceutical Co., Ltd. during the study. Daily care and experimental procedures are conducted by the animal care staff of Shanghai Medix Biopharmaceutical Co., Ltd.

All animal handling and management strictly adhere to the Shanghai Medix Guidelines for the Use and Management of Laboratory Animals.

2. Experimental Materials

2.1 Solvent Formulations and Storage Conditions for Administered Solutions

2.1.1 CAI

Solvent: 100% PEG400

Storage Conditions for Administered Solution: Store at room temperature protected from light. Shelf life: 3 days.

2.1.2 Alectinib

Solvent: 0.5% MC

Storage Conditions for Administered Solution: Store at 4°C protected from light. Shelf life: 7 days.

2.2 Cell Lines

Human lung cancer cell line NCI-H2228 was purchased from the American Type Culture Collection (ATCC, Manassas, VA, USA).

2.3 Media

RPMI 1640 basic medium and fetal bovine serum (FBS) were purchased from GIBCO (Grand Island, NY, USA). Matrigel was purchased from Corning (Corning, NY, USA).

3. Experimental Design

The experimental design is shown in Table 9.

Table 9 Experimental design of therapeutic effect of the drugs *in vivo*

| Groups | Drug | Animal group capacity | Dos age (mg/ kg) | Dosage in concentration | Gro ups | Dr ug | Animal group capacity |
|---|---|---|---|---|---|---|---|
| Control group | Vehi cle | 10 | NA | NA | 10 | P O | QD×21 |
| Comparative Example 4 | Alect inib | 10 | 1 | 0.1 | 10 | P O | QD×21 |
| Comparative Example 5 | CAI | 10 | 20 | 2 | 10 | P O | QD×21 |
| Comparative Example 6 | CAI | 10 | 40 | 4 | 10 | P O | QD×21 |
| Example 4 | CAI | 10 | 20 | 2 | 10 | P O | QD×21 |
| | Alect | | 1 | 0.1 | 10 | P | QD×21 |
| | inib | | | | | O | |

(continued)

| Groups | Drug | Animal group capacity | Dos age (mg/ kg) | Dosage in concentration | Gro ups | Dr ug | Animal group capacity |
|---|---|---|---|---|---|---|---|
| Example 5 | CAI | 10 | 40 | 4 | 10 | P O | QD×21 |
| | Alect inib | | 1 | 0.1 | 10 | P O | QD×21 |

4. Experimental Methods

4.1 Model Establishment

NCI-H2228 cells were cultured in RPMI 1640 medium supplemented with 10% FBS and maintained in a 37°C, 5% $CO_2$ humidified incubator.

Log-phase NCI-H2228 cells were harvested, resuspended in RPMI 1640 basal medium containing 50% Matrigel, and adjusted to a concentration of $2 \times 10^7$ cells/mL. Under aseptic conditions, 0.1 mL of the cell suspension was injected subcutaneously into the right flank of mice at a concentration of $2 \times 10^6$ cells/0.1 mL/mouse.

4.2 Grouping and Administration

When the mean tumor volume reached approximately 150 $mm^3$, animals were randomly grouped based on tumor volume, ensuring inter-group tumor volume variation was less than 10% of the mean.

The grouping day was designated as Day 0, and administration commenced according to animal body weight. During dosing, individual animals exhibiting a body weight loss exceeding 15% compared to Day 0 (BWL ≥ 15%) will be discontinued from treatment until weight recovery (BWL < 15%), after which dosing will resume.

4.3 Weighing and Observation

The same as the therapeutic effect evaluation experiments for Examples 1-2 and Comparative Examples 1-3.

4.4 Endpoint Criteria

The same as the therapeutic effect evaluation experiments for Examples 1-2 and Comparative Examples 1-3.

4.5 Evaluation Indicators

The same as the therapeutic effect evaluation experiments for Examples 1-2 and Comparative Examples 1-3.

5. Statistical Analysis

The same as the therapeutic effect evaluation experiments for Examples 1-2 and Comparative Examples 1-3.

6. Experimental Results

Influence of animal body weight and fatality for different combination of drugs is shown in Table 10.

Table 10 Influence of animal body weight for different combination of drugs

| Groups | Animal group capacity | Drugs | Average body weight (g, Mean ± SEM) | | Body weight change rate Day 21 vs Day 0 | Fatality |
|---|---|---|---|---|---|---|
| | | | Day 0 | Day 21 | | |
| Control group | 10 | Vehicle, PO, QD×21 | 22.04±0.31 | 23.46±0.37 | 6.44% (1.42 g) | 0/10 (Day 21) |

(continued)

| Groups | Animal group capacity | Drugs | Average body weight (g, Mean ± SEM) | | Body weight change rate Day 21 vs Day 0 | Fatality |
|---|---|---|---|---|---|---|
| | | | Day 0 | Day 21 | | |
| Comparative Example 4 | 10 | Alectinib, 1mg/kg, PO, QD×21 | 22.48±0.33 | 22.20±0.41 | -1.13% (-0.28 g) | 0/10 (Day 21) |
| Comparative Example 5 | 10 | CAI, 20mg/kg, PO, QD×21 | 22.08±0.39 | 23.05±0.49 | 4.36% (0.97 g) | 0/10 (Day 21) |
| Comparative Example 6 | 10 | CAI, 40mg/kg, PO, QD×21 | 22.30±0.38 | 22.86±0.37 | 2.55% (0.56 g) | 0/10 (Day 21) |
| Example 4 | 10 | CAI, 20mg/kg, PO, QD×21 + Alectinib, 1mg/kg, PO, QD×21 | 21.67±0.35 | 22.77±0.51 | 1.56% (1.10 g) | 0/10 (Day 21) |
| Example 5 | 10 | CAI, 40mg/kg, PO, QD×21 + Alectinib, 1mg/kg, PO, QD×21 | 21.99±0.30 | 23.81±0.45 | 6.51% (1.82 g) | 0/10 (Day 21) |

As shown in Table 10, no significant inhibitory effect on animal weight gain was observed in the administration of single components CAI in Comparative Examples 4-6 and the administration of combination drugs in Examples 4-5, towards the solvent control group. Compared to Day 0 at the start of the experiment, the average animal body weight increased by 1.56%-6.51% (0.56 g-1.82 g). Alectinib showed a slight inhibition effect on animal body weight. The average animal body weight reduced by 1.13% (-0.28g) compared to Day 0 at the start of the experiment. No drug-related animal deaths or other significant drug-related toxic side effects were observed during the experiment.

Influence on tumor volume, tumor weight and tumor inhibition rate for different combination of drug is shown in Table 11.

Table 11 Influence on tumor volume, tumor weight and tumor inhibition rate for different combination of drugs.

| Groups | Ani mal grou p capa city | Drug s | Tumor volume (mm$^3$, Mean ±SEM) | | Tum or grow th inhib ition rate( %) Day 21 | Relative tumor volume Day 21 | Relativ e tumor prolife ration rate(% ) Day 21 | Tumor weight (g, Mean ±SEM) Day 21 | Tum or inhib ition rate( %) Day 21 |
|---|---|---|---|---|---|---|---|---|---|
| | | | Day 0 | Day 21 | | | | | |
| Contro 1 group | 10 | Vehic le, PO, QD×21 | 163. 3 ± 13.7 7 | 1470.11 ± 224.74 | / | 8.75±0.78 | / | 0.873 ± 0.103 | / |
| Compa rative Examp le 4 | 10 | Alect inib, 1mg/ kg, PO, QD× 21 | 163. 47± 13.2 4 | 863.95± 98.37* | 41.23 | 5.23± 0.32** | 59.77 | 0.444 ± 0.049** | 49.14 |
| Compa rative Examp le 5 | 10 | CAI, 20mg /kg, PO, QD× 21 | 163. 07± 12.9 | 979.25± 64.93* | 33.39 | 6.12±0.29 * | 69.94 | 0.529 ± 0.055* | 39.40 |
| Compa rative Examp le 6 | 10 | CAI, 40mg /kg, PO, QD× 21 | 163. 4 ± 13.2 7 | 970.11± 102.46* | 34.01 | 5.99±0.48 * | 68.46 | 0.498± 0.059* | 42.96 |

(continued)

| Groups | Animal group capacity | Drugs | Tumor volume ($mm^3$, Mean ±SEM) | | Tumor growth inhibition rate(%) Day 21 | Relative tumor volume Day 21 | Relative tumor proliferation rate(%) Day 21 | Tumor weight (g, Mean ±SEM) Day 21 | Tumor inhibition rate(%) Day 21 |
|---|---|---|---|---|---|---|---|---|---|
| | | | Day 0 | Day 21 | | | | | |
| Example 4 | 10 | CAI, 20mg /kg, PO, QD× 21 + Alectinib, 1mg/kg, PO, QD× 21 | 163.13± 12.32 | 639.56± 62.41** && | 56.50 | 3.9±0.21** && | 44.57 | 0.291 ± 0.028** #&& | 66.67 |
| Example 5 | 10 | CAI, 40mg /kg, PO, QD× 21 + Alectinib, 1mg/kg, PO, QD× 21 | 163.36± 13.98 | 539.94± 70.27** # ※※ | 63.27 | 3.5±0.53** #※※ | 40.00 | 0.269 ± 0.041** ##※※ | 69.19 |

Compared to control group, *P<0.05, **P<0.01; compared to Comparative Example 4, #P<0.05 , ##P<0.01; compared to Comparative Example 5, &P<0.05, &&P<0.01; compared to Comparative Example 6, ※P<0.05, ※※P<0.01.

Based on the tumor volume, weight, and tumor inhibition rate data in Table 11, as well as the comparative curves of tumor volume, relative tumor volume, and tumor weight shown in Figures 7 to 9, it can be observed that in Comparative Example 4, the single component Alectinib administered at a dose of 1 mg/kg alone resulted in an average tumor weight of 0.444 ± 0.049 g and a tumor inhibition rate%$TGI_{TW}$ of 49.14%. This represents a significant difference compared to the average tumor weight (0.873 ± 0.103 g) in the solvent control group (P < 0.01). The relative tumor proliferation rate% $T/C_{RTV}$ was 59.77% (P < 0.01).

In Comparative Example 5, the average tumor weight for the single component CAI administered alone at 20 mg/kg was 0.529 ± 0.055 g, and in Comparative Example 6, it was 0.498 ± 0.059 g when administered alone at 40 mg/kg. The tumor inhibition rate % TGITW were 39.40% and 42.96%, respectively, showing no significant difference compared to the solvent control group (P < 0.01); the relative tumor proliferation rates %T/CRTV were 69.94% (P < 0.05) and 68.46% (P < 0.05), respectively.

In Example 4, the combination of CAI at 20 mg/kg and Alectinib at 1 mg/kg resulted in an average tumor weight of 0.291 ± 0.028 g and a tumor inhibition rate%TGITW of 66.67%, showing significant differences compared to the average tumor weights in Comparative Examples 4-5 and the solvent control group (P < 0.05, P < 0.01). The relative tumor growth rate %T/CRTV was 44.57% (P<0.01), showing no significant difference compared to the administration of Alectinib 1 mg/kg alone in Comparative Example 4 (P>0.05), but a significant difference compared to the administration of CAI 20 mg/kg alone in Comparative Example 5 (P<0.01).

In Example 5, the combination of CAI 40 mg/kg and Alectinib 1 mg/kg resulted in an average tumor weight of 0.269 ± 0.041 g and a tumor growth inhibition rate %TGITW of 69.19%, showing significant differences compared to the average tumor weights in Comparative Examples 4, 6, and the solvent control group (P < 0.01). The relative tumor growth rate %T/CRTV was 40.00% (P<0.01), showing significant differences compared to the administration of Alectinib 1 mg/kg alone in Comparative Example 4 (P<0.05) and the administration of CAI 40 mg/kg alone in Comparative Example 6 (P<0.01).

The results indicate that the combination of CAI 20 mg/kg and Alectinib 1 mg/kg in Example 4, as well as the combination of CAI 40 mg/kg and Alectinib 1 mg/kg in Example 5, significantly inhibited the growth and proliferation of human lung cancer NCI-H2228 xenografts in nude mice (P<0.01, P<0.01). Combined with the evaluation of the combination index of drug, the combination of CAI and Alectinib in Examples 4-5 demonstrated an obvious synergistic effect.

The abovementioned detailed description pertains to a specific embodiment of the present invention. This embodiment is not intended to limit the scope of the invention. Any equivalent implementation or modification that does not depart from the scope of the present invention shall be included within the scope of the technical solution of the present

invention.

## Claims

1. A combination drug for anti-lung-cancer, wherein the combination drug comprises a carboxyamidotriazole and an antitumor targeted drug for gene mutations.

2. The combination drug according to claim 1, wherein the antitumor targeted drug is selected from one or more of an antitumor targeted drug for EGFR mutations, an antitumor targeted drug for ALK rearrangements, an antitumor targeted drug for MET mutations, an antitumor targeted drug for ROS1 rearrangements, an antitumor targeted drug for BRAFV600 mutations and an antitumor targeted drug for RET fusions.

3. The combination drug according to claim 2, wherein the antitumor targeted drug for EGFR mutations is selected from at least one of an osimertinib, an icotinib, a gefitinib, an afatinib, an almonertinib or a dacomitinib; the antitumor targeted drug for ALK rearrangements is selected from at least one of a crizotinib, an alectinib, a ceritinib, an ensartinib or a brigatinib.

4. The combination drug according to claim 2, wherein the antitumor targeted drug for MET mutations is a savolitinib; the antitumor targeted drug for ROS1 rearrangements is selected from at least one of a crizotinib or an entrectinib; the antitumor targeted drug for BRAFV600 mutations is selected from at least one of a dabrafenib or a trametinib; the antitumor targeted drug for RET fusions is a pralsetinib.

5. The combination drug according to claim 3, wherein the combination drug comprises the carboxyamidotriazole and the osimertinib.

6. The combination drug according to claim 3, wherein the combination drug comprises the carboxyamidotriazole and the alectinib.

7. The combination drug according to claim 1, wherein a dosage of the carboxyamidotriazole for adults is 1-10mg/kg; a dosage of the antitumor targeting drug for adults is 0.05-10mg/kg.

8. A use of the combination drug according to any one of claims 1-7 for preparing anti-lung-cancer drug.

9. The use according to claim 8, wherein a function of the combination drug comprises one or more of reducing a tumor volume, increasing a tumor growth inhibition rate, decreasing a relative tumor volume, decreasing a relative tumor proliferation rate, or increasing a tumor inhibition rate.

10. A composition, wherein the composition comprises the combination drug according to any one of claims 1-7, the composition is used for preparation of anti-lung-cancer drug, and a dosage form of the composition is selected from any one of oral liquids, softgel capsules, oral suspensions, solid dispersions, capsules, sustained-release formulations, granules, tablets, injections, ointments, patches, or implants.

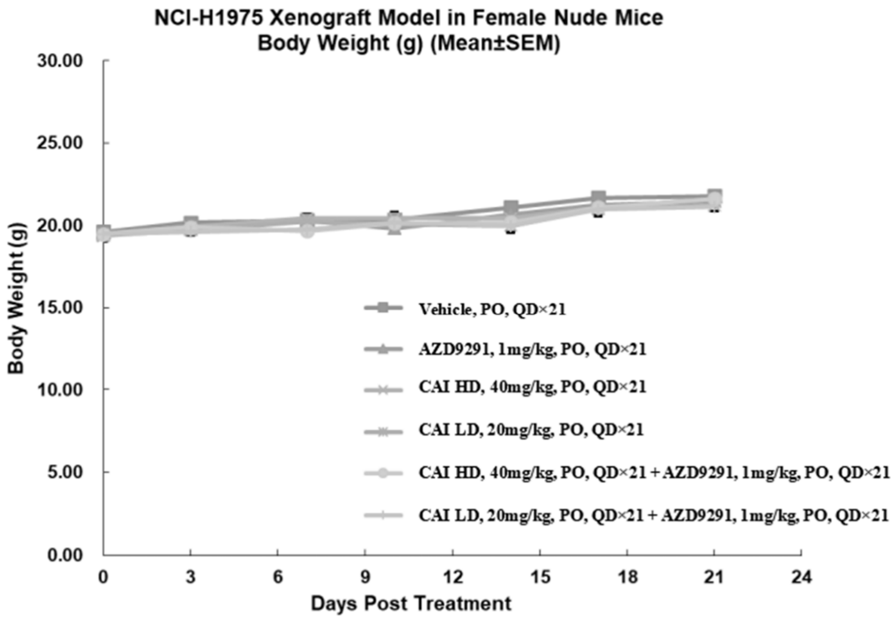
NCI-H1975 Xenograft Model in Female Nude Mice
Body Weight (g) (Mean±SEM)
Body Weight (g)
Days Post Treatment
Vehicle, PO, QD×21
AZD9291, 1mg/kg, PO, QD×21
CAI HD, 40mg/kg, PO, QD×21
CAI LD, 20mg/kg, PO, QD×21
CAI HD, 40mg/kg, PO, QD×21 + AZD9291, 1mg/kg, PO, QD×21
CAI LD, 20mg/kg, PO, QD×21 + AZD9291, 1mg/kg, PO, QD×21

FIG. 1

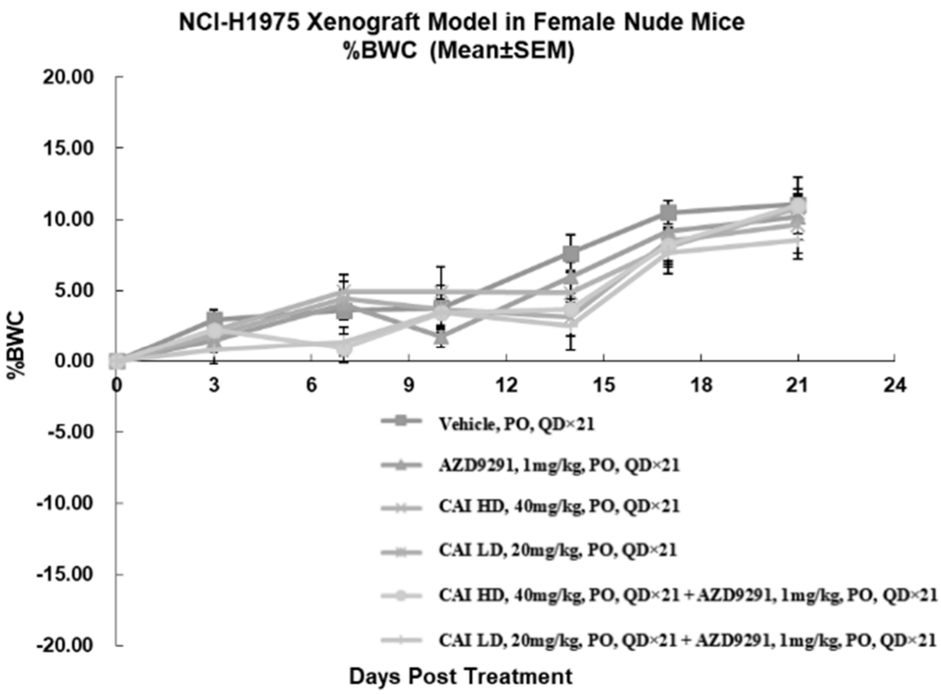
NCI-H1975 Xenograft Model in Female Nude Mice
%BWC (Mean±SEM)
%BWC
Days Post Treatment
Vehicle, PO, QD×21
AZD9291, 1mg/kg, PO, QD×21
CAI HD, 40mg/kg, PO, QD×21
CAI LD, 20mg/kg, PO, QD×21
CAI HD, 40mg/kg, PO, QD×21 + AZD9291, 1mg/kg, PO, QD×21
CAI LD, 20mg/kg, PO, QD×21 + AZD9291, 1mg/kg, PO, QD×21

FIG. 2

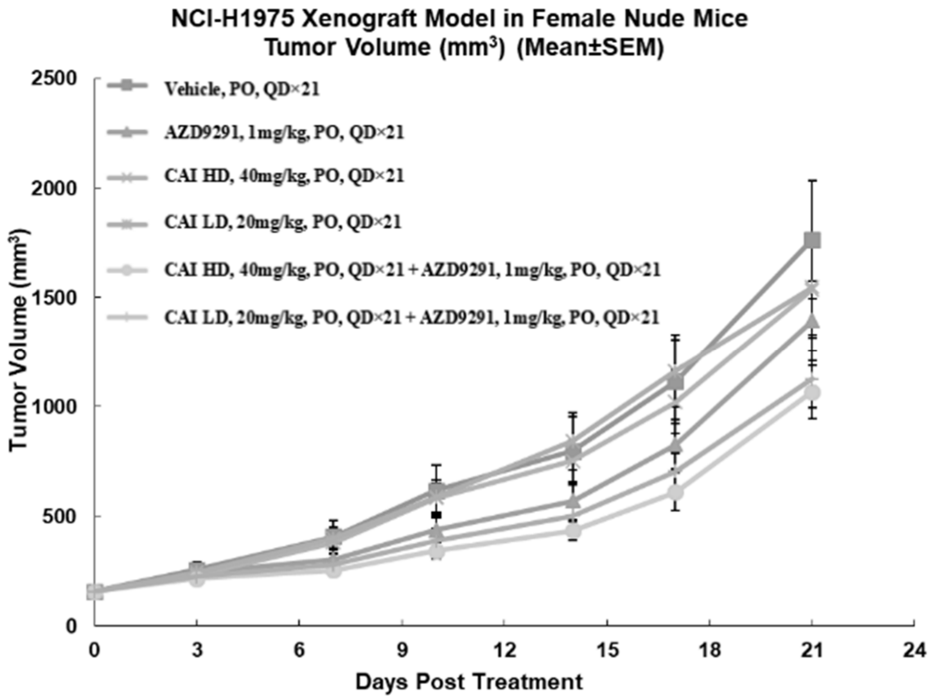
NCI-H1975 Xenograft Model in Female Nude Mice
Tumor Volume (mm³) (Mean±SEM)
Vehicle, PO, QD×21
AZD9291, 1mg/kg, PO, QD×21
CAI HD, 40mg/kg, PO, QD×21
CAI LD, 20mg/kg, PO, QD×21
CAI HD, 40mg/kg, PO, QD×21 + AZD9291, 1mg/kg, PO, QD×21
CAI LD, 20mg/kg, PO, QD×21 + AZD9291, 1mg/kg, PO, QD×21
Tumor Volume (mm³)
Days Post Treatment
0
500
1000
1500
2000
2500
0
3
6
9
12
15
18
21
24

FIG. 3

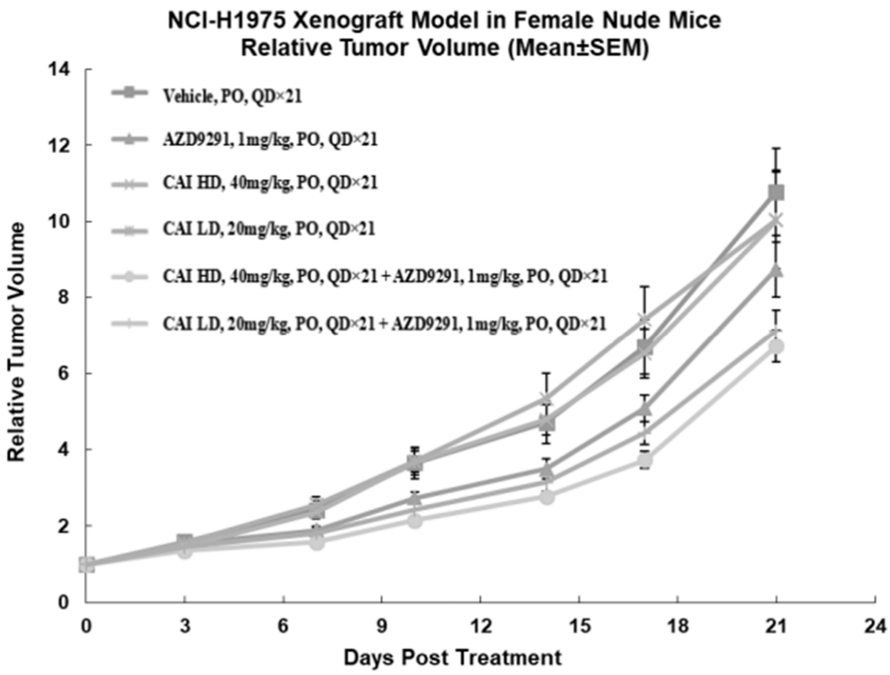
NCI-H1975 Xenograft Model in Female Nude Mice
Relative Tumor Volume (Mean±SEM)
Vehicle, PO, QD×21
AZD9291, 1mg/kg, PO, QD×21
CAI HD, 40mg/kg, PO, QD×21
CAI LD, 20mg/kg, PO, QD×21
CAI HD, 40mg/kg, PO, QD×21 + AZD9291, 1mg/kg, PO, QD×21
CAI LD, 20mg/kg, PO, QD×21 + AZD9291, 1mg/kg, PO, QD×21
Relative Tumor Volume
Days Post Treatment
0
2
4
6
8
10
12
14
0
3
6
9
12
15
18
21
24

FIG. 4

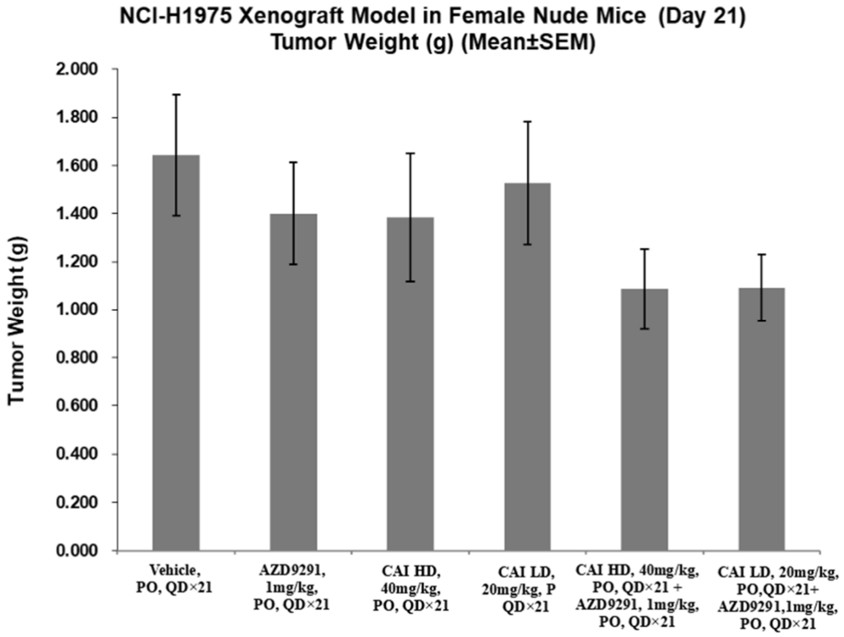
NCI-H1975 Xenograft Model in Female Nude Mice (Day 21)
Tumor Weight (g) (Mean±SEM)
Tumor Weight (g)
2.000
1.800
1.600
1.400
1.200
1.000
0.800
0.600
0.400
0.200
0.000
Vehicle, PO, QD×21
AZD9291, 1mg/kg, PO, QD×21
CAI HD, 40mg/kg, PO, QD×21
CAI LD, 20mg/kg, P QD×21
CAI HD, 40mg/kg, PO, QD×21 + AZD9291, 1mg/kg, PO, QD×21
CAI LD, 20mg/kg, PO,QD×21+ AZD9291,1mg/kg, PO, QD×21

FIG. 5

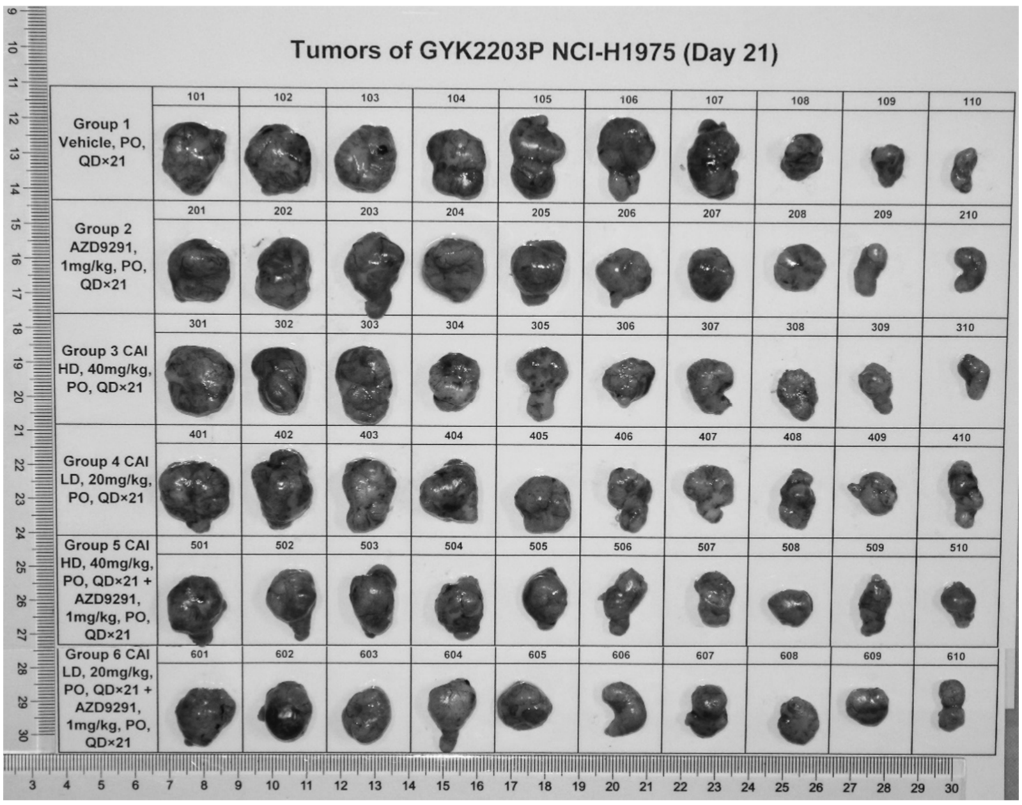
Tumors of GYK2203P NCI-H1975 (Day 21)
Group 1 Vehicle, PO, QD×21
Group 2 AZD9291, 1mg/kg, PO, QD×21
Group 3 CAI HD, 40mg/kg, PO, QD×21
Group 4 CAI LD, 20mg/kg, PO, QD×21
Group 5 CAI HD, 40mg/kg, PO, QD×21 + AZD9291, 1mg/kg, PO, QD×21
Group 6 CAI LD, 20mg/kg, PO, QD×21 + AZD9291, 1mg/kg, PO, QD×21
101 102 103 104 105 106 107 108 109 110
201 202 203 204 205 206 207 208 209 210
301 302 303 304 305 306 307 308 309 310
401 402 403 404 405 406 407 408 409 410
501 502 503 504 505 506 507 508 509 510
601 602 603 604 605 606 607 608 609 610

FIG. 6

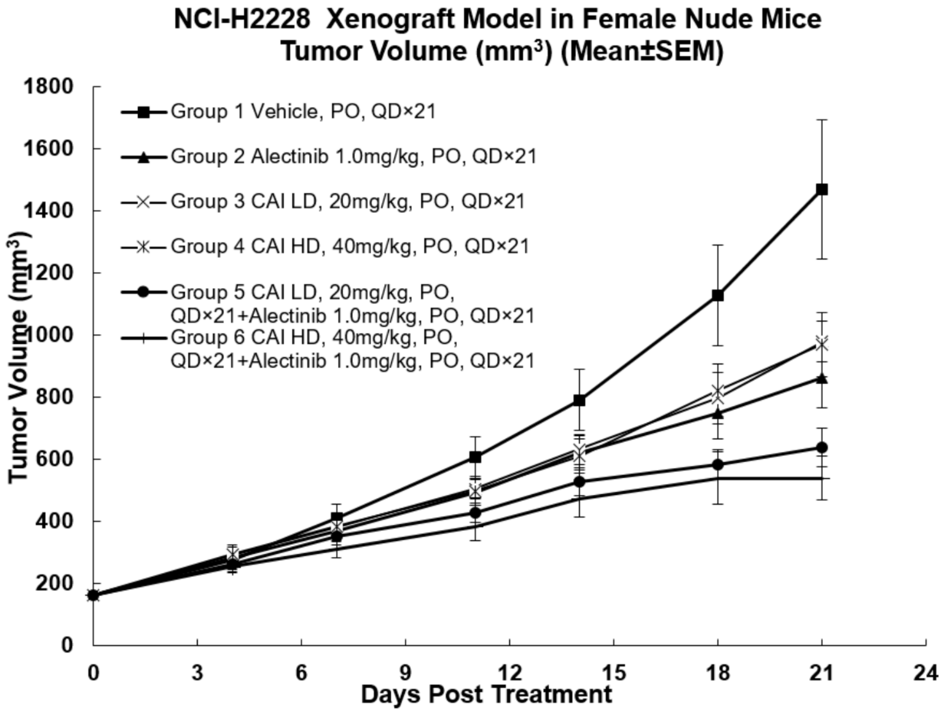
NCI-H2228 Xenograft Model in Female Nude Mice
Tumor Volume (mm3) (Mean±SEM)
Group 1 Vehicle, PO, QD×21
Group 2 Alectinib 1.0mg/kg, PO, QD×21
Group 3 CAI LD, 20mg/kg, PO, QD×21
Group 4 CAI HD, 40mg/kg, PO, QD×21
Group 5 CAI LD, 20mg/kg, PO, QD×21+Alectinib 1.0mg/kg, PO, QD×21
Group 6 CAI HD, 40mg/kg, PO, QD×21+Alectinib 1.0mg/kg, PO, QD×21
Tumor Volume (mm3)
Days Post Treatment

FIG. 7

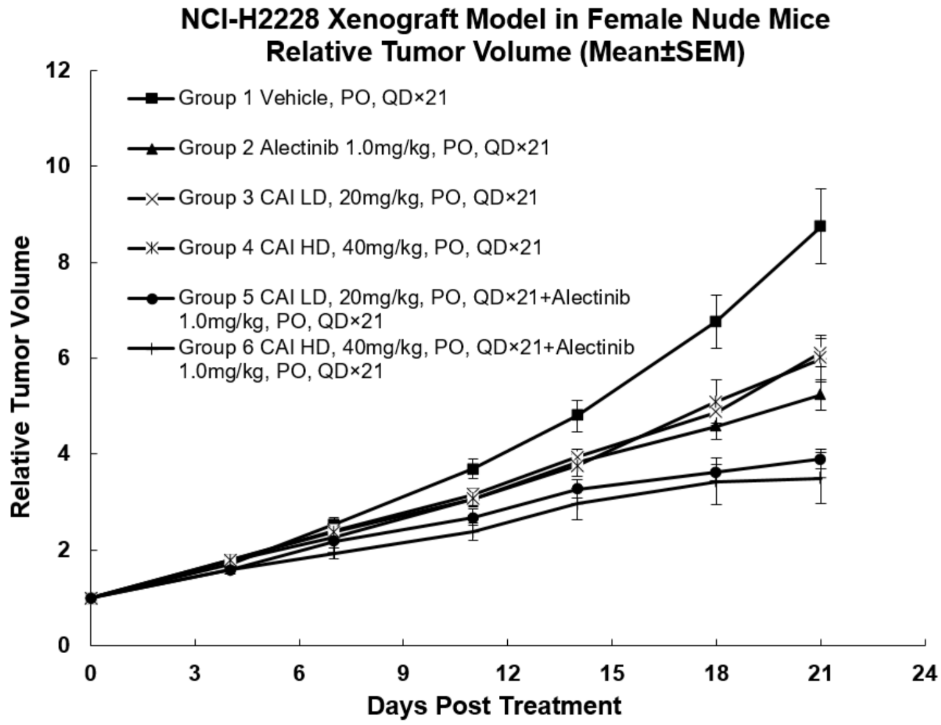
NCI-H2228 Xenograft Model in Female Nude Mice
Relative Tumor Volume (Mean±SEM)
Group 1 Vehicle, PO, QD×21
Group 2 Alectinib 1.0mg/kg, PO, QD×21
Group 3 CAI LD, 20mg/kg, PO, QD×21
Group 4 CAI HD, 40mg/kg, PO, QD×21
Group 5 CAI LD, 20mg/kg, PO, QD×21+Alectinib 1.0mg/kg, PO, QD×21
Group 6 CAI HD, 40mg/kg, PO, QD×21+Alectinib 1.0mg/kg, PO, QD×21
Relative Tumor Volume
Days Post Treatment

FIG. 8

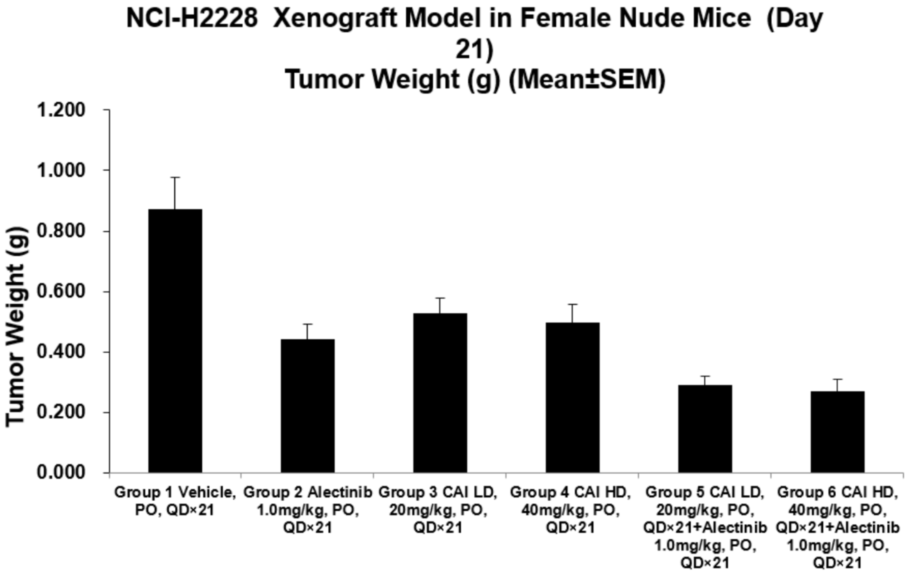
NCI-H2228 Xenograft Model in Female Nude Mice (Day 21)
Tumor Weight (g) (Mean±SEM)
Tumor Weight (g)
1.200
1.000
0.800
0.600
0.400
0.200
0.000
Group 1 Vehicle, PO, QD×21
Group 2 Alectinib 1.0mg/kg, PO, QD×21
Group 3 CAI LD, 20mg/kg, PO, QD×21
Group 4 CAI HD, 40mg/kg, PO, QD×21
Group 5 CAI LD, 20mg/kg, PO, QD×21+Alectinib 1.0mg/kg, PO, QD×21
Group 6 CAI HD, 40mg/kg, PO, QD×21+Alectinib 1.0mg/kg, PO, QD×21

FIG. 9

**INTERNATIONAL SEARCH REPORT**

International application No.
**PCT/CN2024/093033**

**A. CLASSIFICATION OF SUBJECT MATTER**

A61K45/06(2006.01)i; A61K31/4192(2006.01)i; A61K31/506(2006.01)i; A61K31/5377(2006.01)i; A61P35/00(2006.01)i; A61P35/04(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT; WPABSC; ENTXTC; VCN; WPABS; ENTXT; DWPI; VEN; CNKI; CJFD; 百度, BAIDU; 读秀, DUXIU; PubMed; ISI WEB of SCIENCE; STNext Caplus; STNext Registry: 广东银珠医药, 池研生, 胡双华, 许科成, 戎煜, 邢成锋, 寇玉辉, 陈世坚, 羧胺三唑, 奥希替尼, 阿来替尼, 艾乐替尼, 阿雷替尼, 替尼, 肺癌, 基因突变, carboxyamidotriazole, CAI, L651582, NSC 609974, osimertinib, AZD9291, alectinib, RG7853, tinib, lung cancer, lung neoplasm, lung adenocarcinoma, EGFR, ALK, 99519-84-3

**C. DOCUMENTS CONSIDERED TO BE RELEVANT**

| Category* | Citation of document, with indication, where appropriate, of the relevant passages | Relevant to claim No. |
|---|---|---|
| X | CHEN, Chen et al. "Carboxyamidotriazole Synergizes with Sorafenib to Combat Non–Small Cell Lung Cancer through Inhibition of NANOG and Aggravation of Apoptosis" *THE JOURNAL OF PHARMACOLOGY AND EXPERIMENTAL THERAPEUTICS,* Vol. 362, No. 2, 31 August 2017 (2017-08-31), ISSN: 1521-0103, pages 219-229 | 1-2, 4, 7-10 |
| X | CN 105120663 A (TACTICAL THERAPEUTICS, INC.) 02 December 2015 (2015-12-02) claims 3, 6, 9, 10, 12, and 15, and description, paragraphs [0055]-[0056], embodiment 3 | 1-4, 7-10 |
| A | CN 101336889 A (SHANDONG LANJIN PHARMACEUTICALS CO., LTD. et al.) 07 January 2009 (2009-01-07) description, page 3, paragraph 3 to page 5, paragraph 2, and claim 1 | 1-10 |
| A | CN 101669941 A (INSTITUTE OF BASIC MEDICAL SCIENCES, CHINESE ACADEMY OF MEDICAL SCIENCES) 17 March 2010 (2010-03-17) claims 1-15 | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 July 2024** | **25 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/093033**

| C. DOCUMENTS CONSIDERED TO BE RELEVANT | | |
|---|---|---|
| Category* | Citation of document, with indication, where appropriate, of the relevant passages | Relevant to claim No. |
| A | CN 109674788 A (INSTITUTE OF BASIC MEDICAL SCIENCES, CHINESE ACADEMY OF MEDICAL SCIENCES) 26 April 2019 (2019-04-26)<br>claims 1-10 | 1-10 |
| A | CN 109674789 A (INSTITUTE OF BASIC MEDICAL SCIENCES, CHINESE ACADEMY OF MEDICAL SCIENCES) 26 April 2019 (2019-04-26)<br>claims 1-10 | 1-10 |
| A | CN 111643503 A (CHIA TAI TIANQING PHARMACEUTICAL GROUP CO., LTD.) 11 September 2020 (2020-09-11)<br>claims 1-3, 5-7, and 9-10 | 1-10 |
| A | CN 112933087 A (GUANGDONG YINZHU PHARMACEUTICAL TECHNOLOGY CO., LTD.) 11 June 2021 (2021-06-11)<br>claims 1-8 | 1-10 |
| A | CN 1660434 A (INSTITUTE OF BASIC MEDICAL SCIENCES, CHINESE ACADEMY OF MEDICAL SCIENCES) 31 August 2005 (2005-08-31)<br>claims 1-10 | 1-10 |
| A | 武丹威 等 (WU, Danwei et al.). "羧胺三唑联合小剂量地塞米松抗肿瘤作用研究 (The Anti-Tumor Effects of Carboxyamidotriazole and Low Dose Dexamethasone)"<br>*中华临床医师杂志 (电子版) (Chinese Journal of Clinicians (Electronic Edition)),*<br>Vol. 7, No. 7, 01 April 2013 (2013-04-01),<br>ISSN: 1674-0785,<br>pages 2949-2953 | 1-10 |
| A | YAKANTHAMA, T. et al. "Design, Synthesis, and Anticancer Activity of 1,2,3-Triazole Likned Thiazole-1,2-Isoxazole Derivatives"<br>*Russian Journal of General Chemistry,* Vol. 89, No. 12, 26 February 2020 (2020-02-26),<br>ISSN: 1070-3632,<br>pages 2522-2527 | 1-10 |

**INTERNATIONAL SEARCH REPORT**
**Information on patent family members**

International application No.
**PCT/CN2024/093033**

| Patent document cited in search report | | | Publication date (day/month/year) | Patent family member(s) | | | Publication date (day/month/year) |
|---|---|---|---|---|---|---|---|
| CN | 105120663 | A | 02 December 2015 | JP | 2016515619 | A | 30 May 2016 |
| | | | | JP | 6410795 | B2 | 24 October 2018 |
| | | | | AU | 2014248377 | A1 | 03 September 2015 |
| | | | | AU | 2014248377 | B2 | 01 February 2018 |
| | | | | US | 2014294806 | A1 | 02 October 2014 |
| | | | | US | 9089570 | B2 | 28 July 2015 |
| | | | | ZA | 201506037 | B | 29 September 2021 |
| | | | | CA | 2902144 | A1 | 09 October 2014 |
| | | | | CA | 2902144 | C | 09 February 2021 |
| | | | | HK | 1214921 | A1 | 12 August 2016 |
| | | | | KR | 20160026824 | A | 09 March 2016 |
| | | | | EP | 2981169 | A2 | 10 February 2016 |
| | | | | EP | 2981169 | A4 | 14 September 2016 |
| | | | | EP | 2981169 | B1 | 05 May 2021 |
| | | | | IL | 241827 | B | 28 February 2019 |
| | | | | KR | 20210019116 | A | 19 February 2021 |
| | | | | WO | 2014165412 | A2 | 09 October 2014 |
| | | | | WO | 2014165412 | A3 | 29 January 2015 |
| CN | 101336889 | A | 07 January 2009 | | None | | |
| CN | 101669941 | A | 17 March 2010 | | None | | |
| CN | 109674788 | A | 26 April 2019 | | None | | |
| CN | 109674789 | A | 26 April 2019 | | None | | |
| CN | 111643503 | A | 11 September 2020 | | None | | |
| CN | 112933087 | A | 11 June 2021 | | None | | |
| CN | 1660434 | A | 31 August 2005 | WO | 2006076854 | A1 | 27 July 2006 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 200810212286
- CN 109674788 A